# EUROPEAN PATENT SPECIFICATION

(11) **EP 2 783 635 B1**
(45) Date of publication and mention of the grant of the patent: **08.03.2017**
(21) Application number: 13191323.8
(22) Date of filing: 04.11.2013
(51) Int. Cl.: A61B 8/08, A61B 8/00

(54) **Ultrasound system and method of providing direction information of object**
Ultraschallsystem und Verfahren zur Bereitstellung von Richtungsinformationen eines Objekts
Système à ultrasons et un procédé permettant de fournir des informations de direction d'un objet

(30) Priority: 28.03.2013 KR 20130033279
(43) Date of publication of application: 01.10.2014
(73) Proprietor: Samsung Medison Co., Ltd., Gangwon-do 250-870 (KR)
(72) Inventor: Lee, Jun-kyo, Hongcheon-gun, Gangwon-do (KR)
(74) Representative: Lorenz, Markus

(56) References cited:
- JP-A- H07 116 159
- JP-A- 2000 175 910
- JP-A- 2010 187 987
- US-A1- 2010 125 201

## Description

### BACKGROUND

### 1. Field

One or more embodiments of the present invention relate to an ultrasound system, and more particularly, to an ultrasound system and method of providing direction information of an object (for example, a fetus).

### 2. Description of the Related Art

An ultrasound system has noninvasive and nondestructive characteristics, and thus is widely used in the medical field to obtain internal information of an object. The ultrasound system provides a high-resolution image of internal tissue of an object to a doctor in real time without requiring a surgical operation that directly incises and via which the object is observed, and thus is very importantly used in the medical field.

The ultrasound system transmits an ultrasound signal to an object, receives the ultrasound signal (i.e., an ultrasound echo signal) reflected from the object, and generates a two-dimensional (2D) or three-dimensional (3D) ultrasound image by using the received ultrasound echo signal.

In fetuses, a risk of a cardiac anomaly is determined with respect to a position of a heart. For example, it is reported that when a heart of a fetus is normally located, a risk of a cardiac anomaly is about 1%, but when a heart of a fetus is located at the right, namely, when dextrocardia, the risk of cardiac anomaly is about 95%. Therefore, it is important to accurately determine a heart position of a fetus, for predicting the risk of cardiac anomaly. However, since a fetus freely moves in a uterus, it is difficult for a skilled user to accurately determine a direction of the fetus in an image, and due to this, an error occurs in predicting the risk of cardiac anomaly based on a position of a heart.

The purpose of the JP H07 116159 A is to provide an ultrasonograph to inform the direction of a fetus, and the head and heart position of the fetus, by irradiating an ultrasonic wave on the stomach of a pregnant woman, obtaining the ultrasonic image of the fetus, and displaying the outline image of the fetus synthesized with the ultrasonic image. This is solved by the supply of a driving voltage with a constant frequency to a transmitter/receiver circuit transmitting an ultrasonic wave into the stomach of a pregnant woman repeatedly from a head-swinging vibrator. The ultrasonic wave reflects at a sharply changing point of the acoustic impedance in the stomach and the reflected wave is received at the vibrator. This received signal is sequentially stored in the storage space of a DSC, whose output signal is displayed on a TV monitor through an adder. And from among various fetus body marks registered on a body mark table, a body mark selected by an operator according to the pregnant term and the direction of the fetus can be picked up by operating the input device, and is synthesized with the ultrasonic image at the adder under the control by a CPU. The synthesized image is displayed on the monitor.

The problem to be solved by the JP 2010 187987 A is to provide an ultrasonic diagnostic apparatus, reducing the time and labor taken to display fetal form information, and used in prenatal examination. This problem is solved by a form information storage part storing a plurality of week numbers in pregnancy and a plurality of form information pieces following the fetal form corresponding to the plurality of week numbers in pregnancy in association with each other. An ultrasonic probe transmits and receives an ultrasonic wave. An image generating part generates data of an ultrasonic image related to the fetus based on an echo signal from the ultrasonic probe. A form information selecting part selects specific form information related to the week number in pregnancy of the fetus among the plurality of form information pieces. A display part displays a selected specific form information and an ultrasonic image. A form information adjusting part adjusts at least the magnitude of specific form information according to the measured values of the fetus measured on the fetal reflection on the ultrasonic image.

### SUMMARY

One or more embodiments of the present invention include an ultrasound system and method that add direction information of a fetus to an ultrasound image, thereby accurately determining a position of an object of interest (for example, a heart) of the fetus in the ultrasound image.

Additional aspects will be set forth in part in the description which follows and, in part, will be apparent from the description, or may be learned by practice of the presented embodiments.

According to one or more embodiments of the present invention, an ultrasound system of claim 1 includes: an ultrasound data acquirer that transmits an ultrasound signal to an object including a fetus, and acquires ultrasound data by receiving an ultrasound echo signal reflected from the object; a processor that generates an ultrasound image with the ultrasound data; a display that displays the ultrasound image on a screen; and a user input unit that receives a user command for setting position information of the fetus corresponding to a posture of the fetus based on the ultrasound image, wherein the processor sets direction information, indicating the posture of the fetus, on the screen in response to the user command.

The processor detects a central point of the ultrasound image, sets a width central line and a height central line with respect to the central point on the ultrasound image, detect a position of the OOI based on the direction information of the fetus, the width central line, and the height central line, and generate the position information of the OOI based on the detected position of the OOI.

The ultrasound system may further include a storage that stores a plurality of icons indicating a plurality of fetus postures and direction information of a fetus corresponding to each of the plurality of icons.

The user command may include: a first user command for displaying the plurality of icons on the screen of the display; and a second user command for selecting one icon from among the plurality of icons.

The processor may display the plurality of icons on the screen of the display in response to the first user command, and set direction information, corresponding to the selected icon, on the screen of the display in response to the second user command.

The direction information of the fetus includes at least one of an anterior, posterior, left, and right of the fetus.

The processor detects an object of interest (OOI) from the ultrasound image of the object displayed on the screen with the direction information of the fetus set thereon, and generate position information of the detected OOI based on the direction information of the fetus which is set on the screen.

The processor may generate additional information indicating a degree of proximity by which the OOI approaches from the central point to the position information of the fetus, based on the direction information of the fetus.

The OOI may include a heart of the fetus.

According to one or more embodiments of the present invention, a method of providing position information of an object in an ultrasound system of claim 8 includes: transmitting an ultrasound signal to an object including a fetus, and acquiring ultrasound data by receiving an ultrasound echo signal reflected from the object; generating an ultrasound image with the ultrasound data; displaying the ultrasound image on a screen; receiving a user command for setting position information of the fetus corresponding to a posture of the fetus based on the ultrasound image; and setting direction information, indicating the posture of the fetus, on the screen in response to the user command.

The method further includes: detecting a central point of the ultrasound image; setting a width central line and a height central line with respect to the central point on the ultrasound image; detecting a position of the OOI based on the direction information of the fetus, the width central line, and the height central line; and generating the position information of the OOI based on the detected position of the OOI.

The method may further include storing a plurality of icons indicating a plurality of fetus postures and direction information of a fetus corresponding to each of the plurality of icons.

The user command may include: a first user command for displaying the plurality of icons on the screen; and a second user command for selecting one icon from among the plurality of icons.

The method further includes:
displaying the plurality of icons on the screen in response to the first user command; and setting direction information, corresponding to the selected icon, on the screen in response to the second user command.

The direction information of the fetus may include at least one of an anterior, posterior, left, and right of the fetus.

The method further includes:
detecting an object of interest (OOI) from the ultrasound image of the object displayed on the screen with the direction information of the fetus set thereon; and generating position information of the detected OOI based on the direction information of the fetus which is set on the screen.

The method may further include generating additional information indicating a degree of proximity by which the OOI approaches from the central point to the position information of the fetus, based on the direction information of the fetus.

The OOI may include a heart of the fetus.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a configuration of an ultrasound system according to an embodiment of the present invention;
FIG. 2 is a block diagram illustrating a configuration of an ultrasound data acquirer according to an embodiment of the present invention;
FIG. 3 is an exemplary diagram illustrating examples of a plurality of icons showing a shape of a fetus based on a posture of the fetus, according to an embodiment of the present invention;
FIG. 4 is a flowchart of a procedure of setting direction information of a fetus, according to an embodiment of the present invention;
FIG. 5 is an exemplary diagram illustrating an example of a screen in which direction information of a fetus corresponding to each of a plurality of icons is set;
FIG. 6 is a flowchart of a procedure of determining a position of a heart in an ultrasound image of a fetus, according to an embodiment of the present invention; and
FIG. 7 is an exemplary diagram illustrating additional information according to an embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Hereinafter, embodiments of the present invention will be described in detail with reference to the accompanying drawings.

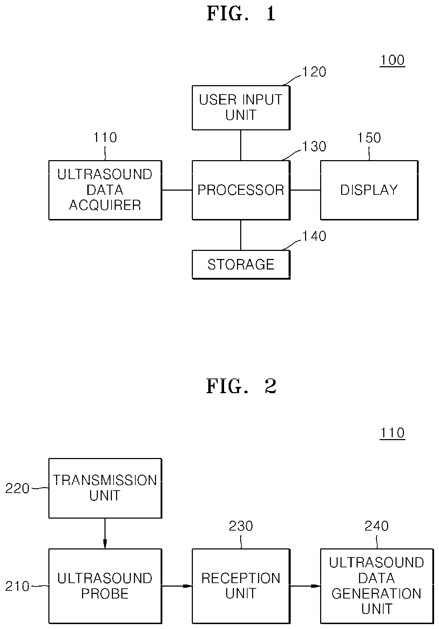

FIG. 1 is a block diagram illustrating a configuration of an ultrasound system 100 according to an embodiment of the present invention. Referring to FIG. 1, the ultrasound system 100 includes an ultrasound data acquirer 110. The ultrasound data acquirer 110 transmits an ultrasound signal to an object, and receives the ultrasound signal (namely, an ultrasound echo signal) reflected from the object to acquire ultrasound data. In an embodiment, the object includes a fetus.

FIG. 2 is a block diagram illustrating a configuration of the ultrasound data acquirer 110 according to an embodiment of the present invention. Referring to FIG. 2, the ultrasound data acquirer 110 includes an ultrasound probe 210.

The ultrasound probe 210 includes a plurality of transducer elements (not shown) that convert between an electrical signal and an ultrasound signal. The ultrasound probe 210 transmits an ultrasound signal to an object, and receives an ultrasound echo signal reflected from the object to output an electrical signal (hereinafter, referred to as a reception signal). The reception signal is an analog signal. The ultrasound probe 210 includes a convex probe, a linear probe, and a 3D probe.

The ultrasound data acquirer 110 further includes a transmission unit 220. The transmission unit 220 controls transmission of an ultrasound signal. Also, the transmission unit 220 includes a plurality of pulsers (not shown), and generates a plurality of pulse signals. The transmission unit 220 delays, for a certain time, a pulse signal output from each of the plurality of pulsers in consideration of a transducer element and a focusing point, thereby outputting a transmission pulse signal having a certain transmission profile. The ultrasound probe 210 generates and outputs an ultrasound signal in response to the transmission pulse signal supplied from the transmission unit 220. An ultrasound beam output from the ultrasound probe 210 may form and output a beam in a certain direction (for example, a scan line) according to a profile of a transmission pulse. Also, the ultrasound probe 210 receives an ultrasound echo signal reflected from an object to output an electrical reception signal.

The ultrasound data acquirer 110 further includes a reception unit 230. The reception unit 230 analog-digital-converts the reception signal supplied from the ultrasound probe 210 to generate a digital signal. Also, the reception unit 230 performs reception beamforming on the digital signal in consideration of the transducer element and the focusing point to generate a reception focusing signal. In an embodiment, the reception beamforming by the reception unit 230 may be performed by using an application specific integrated circuit (ASIC) in which a physical delay means such as a resistor is integrated, or may be performed by using a digital signal processor (DSP) as a software algorithm.

The ultrasound data acquirer 110 further includes an ultrasound data generation unit 240. The ultrasound data generation unit 240 generates ultrasound data corresponding to an ultrasound image by using the reception focusing signal supplied from the reception unit 230. The ultrasound data includes radio frequency (RF) data. However, the ultrasound data is not limited thereto. Also, the ultrasound data acquirer 110 may perform various signal processing (for example, gain adjustment, etc.), which is necessary to generate the ultrasound data, on the reception focusing signal. In an embodiment, the ultrasound data acquirer 110 may be implemented with one or more DSPs.

Referring again to FIG. 1, the ultrasound system 100 further includes a user input unit 120. The user input unit 120 receives various user commands for driving the ultrasound system 100 from a user. In an embodiment, the user commands include a first user command for setting direction information of a fetus in a uterus. In an embodiment, the direction information of the fetus is information indicating at least one of an anterior (Ant), posterior (Post), left (Lt), and right (Rt) of the fetus in an ultrasound image which the user acquires from an object. The user input unit 120 includes at least one of a control panel, a trackball, a mouse, a keyboard, and a touch screen.

The ultrasound system 100 further includes a processor 130. The processor 130 is connected to the ultrasound data acquirer 110 and the user input unit 120. The processor 130 includes a central processing unit (CPU), a microprocessor, or a graphics processing unit (GPU). The processor 130 receives ultrasound data from the ultrasound data generation unit 240, and performs various image processing, such as scan conversion, rendering, etc., on the ultrasound data to generate an ultrasound image.

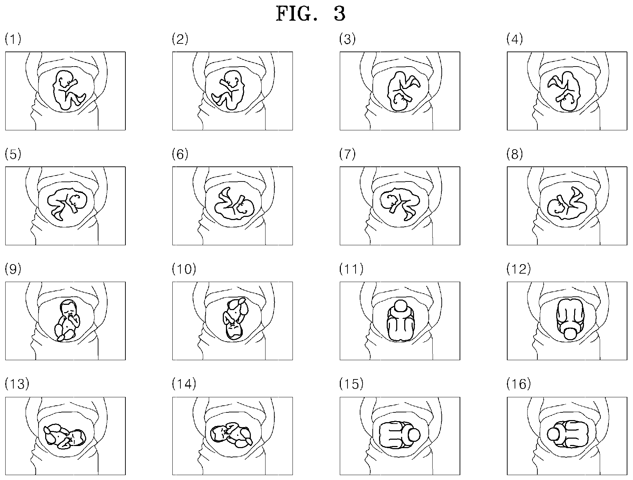

The ultrasound system 100 further includes a storage 140. The storage 140 may store the ultrasound data generated by the ultrasound data generation unit 240. Also, the storage 140 may store the ultrasound image generated by the processor 130. In an embodiment, the storage 140 stores a plurality of icons showing a shape of a fetus based on a posture which the fetus makes in a uterus. FIG. 3 is an exemplary diagram illustrating examples of a plurality of icons showing a shape of a fetus based on a posture of the fetus, according to an embodiment of the present invention. In an embodiment, the storage 140 is described as storing the plurality of icons showing the posture of the fetus, but an image showing the posture of the fetus is not limited thereto. In an embodiment, the storage 140 further stores direction information of the fetus corresponding to each of the plurality of icons. For example, the storage 140 may store a plurality of templates schematically showing the posture of the fetus. The storage 140 includes at least one storage medium of a flash memory, a hard disk, a multimedia card micro, a card-type memory such as a secure digital (SD) or extreme digital (XD) memory, random access memory (RAM), static random access memory (SRAM), read-only memory (ROM), electrically erasable programmable read-only memory (EEPROM), programmable read-only memory (PROM), a magnetic memory, a magnetic disc, and an optical disc. The ultrasound system 100 may operate web or cloud storage for performing a storage function of a memory on a network such as the internet.

The ultrasound system 100 further includes a display 150. The ultrasound image generated by the processor 130 is transferred to the display 150, and displayed on a screen. Also, in response to a user command input through the user input unit 120, the processor 130 may read the ultrasound image stored in the storage 140, and transfer the read ultrasound image to the display 150, thereby allowing the ultrasound image to be displayed. In an embodiment, in response to the first user command input through the user input unit 120, the processor 130 reads the plurality of icons stored in the storage 140, and allows the read icons to be displayed by the display 150. The display 150 includes a liquid crystal display (LCD), a cathode-ray tube (CRT) display, an organic light-emitting diode (OLED) display, or the like to display the ultrasound image.

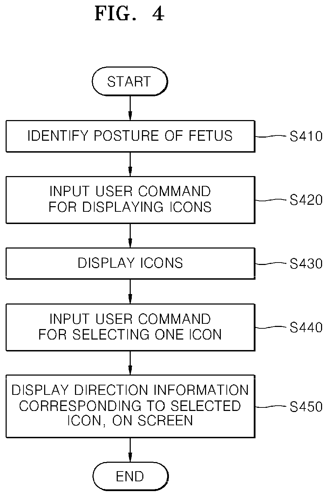

FIG. 4 is a flowchart of a procedure of setting direction information of a fetus, according to an embodiment of the present invention. Referring to FIG. 4, a user scans a fetus according to a scanning protocol to identify a posture of the fetus in operation S410. In an embodiment, the scanning protocol is a scanning protocol in which a user identifies the feet of a fetus while performing ultrasound scanning, and then performs the ultrasound scanning from the feet to the legs and toward the head. However, the scanning protocol is not limited thereto, and all scanning protocols enabling a user to identify a posture of a fetus may be used. A user may perform ultrasound scanning according to a certain scanning protocol to identify a posture of a fetus in consideration of a spine position, a head position, etc. of the fetus in a currently displayed ultrasound image.

When the posture of the fetus is identified, the user inputs the first user command for reading the plurality of icons stored in the storage 140 and displaying the read icons, through the user input unit 120 in operation S420. The processor 130 searches the storage 140 in response to the first user command, reads the plurality of icons, and transfers the read icons to the display 150 to allow the display 150 to display the icons on a screen in operation S430.

The user receives a user command through the user input unit 120 to select an icon corresponding to a current posture of the fetus from among the plurality of icons in operation S440. Here, the user command may allow the user to move a cursor displayed on a screen of the display 150, click a corresponding icon, and give a number to each of the plurality of icons to select the corresponding icon, or touch the corresponding icon on a touch screen. However, the input of the user command is not limited thereto.

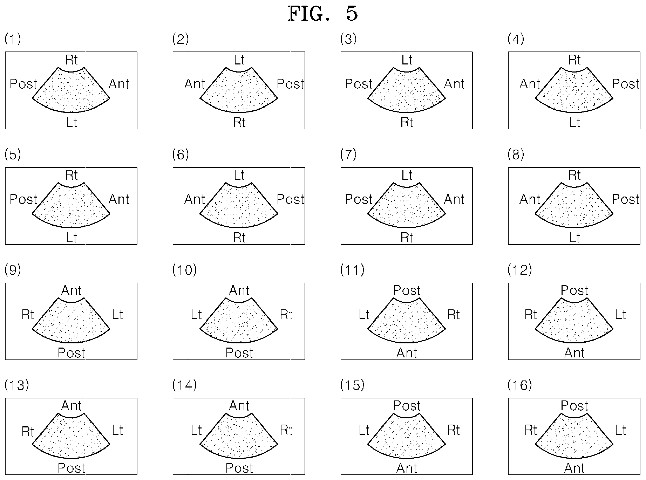

When the icon corresponding to the current posture of the fetus is selected from among the plurality of icons, the processor 130 reads direction information of the fetus from the storage 140, and allows the display 150 to display position information (i.e., direction information indicating an anterior (Ant), posterior (Post), left (Lt), and right (Rt) of the fetus) of the fetus corresponding to the selected icon at a certain position of the screen in operation S450. FIG. 5 is an exemplary diagram illustrating an example of a screen in which direction information of a fetus corresponding to each of a plurality of icons is set. For example, when the user selects an icon number 1 from among the plurality icons of FIG. 3, a first image of FIG. 5 is displayed on the screen as the direction information of the fetus.

In an embodiment, selecting the direction information of the fetus has been described above as a method that selects one icon corresponding to the posture of the fetus from among the plurality of icons, but is not limited thereto. For example, a plurality of markers indicating at least one of an anterior, posterior, left, and right of a fetus may be provided, and a user may select a marker corresponding to a current posture of the fetus from among the plurality of markers to set direction information of the fetus. Also, in another embodiment, by providing a text input window on a screen displaying an ultrasound image, a user may directly input direction information of a fetus.

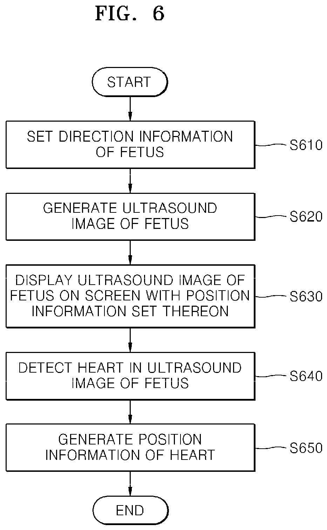

FIG. 6 is a flowchart of a procedure of determining a position of a heart in an ultrasound image of a fetus, according to an embodiment of the present invention. Referring to FIG. 6, a user performs ultrasound scanning on a fetus according to a scanning protocol to determine a posture of the fetus, and sets direction information based on the posture of the fetus on a screen of the display 150 in operation S610. Subsequently, the processor 130 generates an ultrasound image of the fetus by using ultrasound data supplied from the ultrasound data acquirer 110 in operation S620. In an embodiment, the ultrasound image includes a 2D image, namely, a B mode image. However, the ultrasound image is not limited thereto. The ultrasound image generated by the processor 130 is displayed on the screen of the display 150, on which position information of the fetus is set in operation S630.

The processor 130 performs image processing on the ultrasound image with the direction information of the fetus set thereon to detect an object of interest (OOI), for example, a heart, in operation S640. The heart may be detected by various known image processing, and thus, a detailed description thereof is not provided in the embodiment. For example, the image processing may use a method that detects an OOI based on pre-learned data, a method that detects an OOI by acquiring a position value of a moving part in continuous ultrasound images, a method that detects an OOI based on a probability distribution of image brightness, and a method that detects an OOI based on a user input.

The processor 130 generates position information of the heart of the fetus, which is detected from the ultrasound image on the basis of the position information of the fetus that is set on the screen in operation S650.

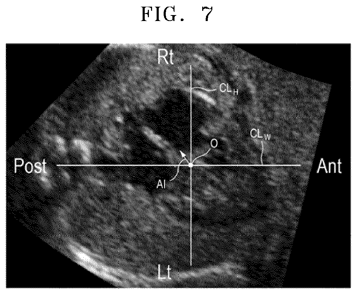

For example, as illustrated in FIG. 7, the processor 130 detects a central point O of the ultrasound image. The central point O may be detected by various known methods, and thus, a detailed description thereof is not provided in the embodiment. The processor 130 sets a width central line CLW and a height central line CLH with respect to the detected central point O, in the ultrasound image. The processor 130 determines a position (Lt, Ant) of the heart of the fetus in the ultrasound image, on the basis of the direction information (Lt, Rt, Ant, and Post), the width central line CLW, the height central line CLH which are set in the ultrasound image, and generates position information of the heart on the basis of the direction information of the fetus.

Alternatively, the processor 130 may generate additional information indicating a degree of proximity by which the heart approaches each of the anatomical positions (Lt, Rt, Ant, and Post), on the basis of the anatomical positions (Lt, Rt, Ant, and Post) of the fetus. For example, as illustrated in FIG. 7, the processor 130 generates additional information Al indicating a degree of proximity by which the heart of the fetus (for example, ventricles of the heart of the fetus) approaches from the central point O of the ultrasound image to each of the anatomical positions (Lt, Rt, Ant, and Post), on the basis of the position information (Lt, Rt, Ant, and Post) of the fetus that is set in the ultrasound image.

According to the embodiments of the present invention, the ultrasound system displays direction information of a fetus on a screen displaying an ultrasound image to enable a user to determine a position of a body organ such as a heart of the fetus, thus decreasing an error. Also, the ultrasound system provides an OOI (i.e., position information of the heart) of the fetus, and thus enables the user to easily determine a risk of a cardiac anomaly.

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the scope of the present invention as defined by the following claims.

## Claims

1. An ultrasound system (100) comprising:
an ultrasound data acquirer (110) adapted to transmit an ultrasound signal to an object including a fetus, and acquire ultrasound data by receiving an ultrasound echo signal reflected from the object;
a processor (130) adapted to generate an ultrasound image with the ultrasound data;
a display (150) adapted to display the ultrasound image on a screen; and
a user input unit (120) adapted to receive a user command for setting position information of the fetus corresponding to a posture of the fetus based on the ultrasound image,
wherein the processor (130) is adapted to set direction information, indicating the posture of the fetus and including at least one of an anterior, posterior, left and right of the fetus, on the screen in response to the user command, **characterised in that** the processor is adapted to detect an object of interest (OOI) from the ultrasound image of the object displayed on the screen with the direction information of the fetus set thereon, detect a central point of the ultrasound image, set a width central line and a height central line with respect to the central point on the ultrasound image, detect a position of the OOI based on the direction information of the fetus, the width central line, and the height central line and generate position information of the detected OOI based on the detected position of the OOI.

2. The ultrasound system (100) of claim 1, further comprising a storage (140) adapted to store a plurality of icons indicating a plurality of fetus postures and direction information of a fetus corresponding to each of the plurality of icons.

3. The ultrasound system (100) of claim 2, wherein the user command includes:
a first user command for displaying the plurality of icons on the screen of the display (150); and
a second user command for selecting one icon from among the plurality of icons.

4. The ultrasound system (100) of claim 3, wherein the processor (130) is adapted to display the plurality of icons on the screen of the display (150) in response to the first user command, and set direction information, corresponding to the selected icon, on the screen of the display (150) in response to the second user command.

5. The ultrasound system (100) of claim 1, wherein the processor (130) is adapted to generate additional information indicating a degree of proximity by which the OOI approaches from the central point to the position information of the fetus, based on the direction information of the fetus.

6. The ultrasound system (100) of claim 1, wherein the OOI includes a heart of the fetus.

7. A method of providing position information of an object using the ultrasound system (100) of claim 1, the method comprising:
transmitting an ultrasound signal to an object including a fetus, and acquiring ultrasound data by receiving an ultrasound echo signal reflected from the object;
generating an ultrasound image with the ultrasound data;
displaying the ultrasound image on a screen;
receiving a user command for setting position information of the fetus corresponding to a posture of the fetus based on the ultrasound image;
and setting direction information, indicating the posture of the fetus and including at least one of an anterior, posterior, left and right of the fetus, on the screen in response to the user command;
**characterised in** detecting an object of interest (OOI) from the ultrasound image of the object displayed on the screen with the direction information of the fetus set thereon;
detecting a central point of the ultrasound image, setting a width central line and a height central line with respect to the central point on the ultrasound image;
detecting a position of the OOI based on the direction information of the fetus, the width central line, and the height central line; and
generating position information of the detected OOI based on the detected position of the OOI.

8. The method of claim 7, further comprising storing a plurality of icons indicating a plurality of fetus postures and direction information of a fetus corresponding to each of the plurality of icons.

9. The method of claim 8, wherein the user command includes:
a first user command for displaying the plurality of icons on the screen; and
a second user command for selecting one icon from among the plurality of icons.

10. The method of claim 9, further comprising:
displaying the plurality of icons on the screen in response to the first user command; and
setting direction information, corresponding to the selected icon, on the screen in response to the second user command.

## Patentansprüche

1. Ultraschallsystem (100), welches Folgendes aufweist:
eine Ultraschalldatenerlangungseinrichtung (110), die dafür vorgesehen ist, ein Ultraschallsignal zu einem Objekt einschließlich eines Fötus zu übertragen und durch Empfangen eines von dem Objekt reflektierten Ultraschallechosignals Ultraschalldaten zu erlangen;
einen Prozessor (130), der dafür vorgesehen ist, ein Ultraschallbild mit den Ultraschalldaten zu erzeugen;
eine Anzeige (150), die dafür vorgesehen ist, das Ultraschallbild auf einem Bildschirm anzuzeigen; und
eine Benutzereingabeeinheit (120), die dafür vorgesehen ist, einen Benutzerbefehl zum Festlegen von Positionsinformationen des Fötus entsprechend einer Haltung bzw. Lage des Fötus basierend auf dem Ultraschallbild zu empfangen, wobei der Prozessor (130) dafür vorgesehen ist, eine Richtungsinformation festzulegen, welche die Haltung bzw. Lage des Fötus anzeigt und wenigstens eines eines anterior, posterior, links und rechts des Fötus auf dem Bildschirm als Reaktion auf den Benutzerbefehl beinhaltet,
**dadurch gekennzeichnet, dass**
der Prozessor dafür vorgesehen ist, ein Objekt von Interesse (OOI) von dem Ultraschallbild des auf dem Bildschirm angezeigten Objekts mit der darauf enthaltenen Richtungsinformation des Fötus zu detektieren, einen zentralen Punkt des Ultraschallbilds zu detektieren, eine Breitenmittellinie und eine Höhenmittellinie in Bezug auf den mittleren bzw. zentralen Punkt auf dem Ultraschallbild festzulegen, eine Position des OOI basierend auf der Richtungsinformation des Fötus, der Breitenmittellinie und der Höhenmittellinie zu detektieren und Positionsinformationen des detektierten OOI basierend auf der detektierten Position des OOI zu erzeugen.

2. Ultraschallsystem (100) nach Anspruch 1, welches des Weiteren einen Speicher (140) aufweist, der dafür vorgesehen ist, eine Vielzahl von Symbolen zu speichern, die eine Vielzahl von Fötushaltungen bzw. -lagen und Richtungsinformationen eines Fötus anzeigen, die jeder der Vielzahl von Symbolen entsprechen.

3. Ultraschallsystem (100) nach Anspruch 2, wobei der Benutzerbefehl Folgendes beinhaltet:
einen ersten Benutzerbefehl zum Anzeigen der Vielzahl von Symbolen auf dem Bildschirm der Anzeige (150); und
einen zweiten Benutzerbefehl zum Auswählen eines Symbols aus der Vielzahl von Symbolen.

4. Ultraschallsystem (100) nach Anspruch 3, wobei der Prozessor (130) dafür vorgesehen ist, die Vielzahl von Symbolen auf dem Bildschirm der Anzeige (150) als Reaktion auf den ersten Benutzerbefehl anzuzeigen und Richtungsinformationen entsprechend dem ausgewählten Symbol auf dem Bildschirm der Anzeige (150) als Reaktion auf den zweiten Benutzerbefehl festzulegen.

5. Ultraschallsystem (100) nach Anspruch 1, wobei der Prozessor (130) dafür vorgesehen ist, zusätzliche Informationen zu erzeugen, die einen Grad der Nähe anzeigen, um den sich der OOI von dem zentralen Punkt der Positionsinformationen des Fötus basierend auf den Richtungsinformationen des Fötus annähert.

6. Ultraschallsystem (100) nach Anspruch 1, wobei der OOI ein Herz des Fötus beinhaltet.

7. Verfahren zum Erzeugen von Positionsinformationen eines Objekts unter Verwendung des Ultraschallsystems (100) nach Anspruch 1, wobei das Verfahren Folgendes aufweist:
Übertragen eines Ultraschallsignals zu einem Objekt einschließlich eines Fötus und Erlangen von Ultraschalldaten durch Empfangen eines von dem Objekt reflektierten Ultraschallechosignals;
Erzeugen eines Ultraschallbilds mit den Ultraschalldaten;
Anzeigen des Ultraschallbilds auf einem Bildschirm;
Empfangen eines Benutzerbefehls zum Festlegen von Positionsinformationen des Fötus entsprechend einer Haltung bzw. Lage des Fötus basierend auf dem Ultraschallbild;
und Festlegen von Richtungsinformationen, welche die Haltung bzw. Lage des Fötus anzeigen und wenigstens eines eines anterior, posterior, links und rechts des Fötus beinhalten, auf dem Bildschirm als Reaktion auf den Benutzerbefehl; **gekennzeichnet durch**
Detektieren eines Objekts von Interesse (OOI) von dem Ultraschallbild des Objekts, das auf dem Bildschirm mit der darauf enthaltenen Richtungsinformation des Fötus angezeigt wird;
Detektieren eines zentralen Punkts des Ultraschallbilds, Festlegen einer Breitenmittellinie und einer Höhenmittellinie in Bezug auf den mittleren bzw. zentralen Punkt auf dem Ultraschallbild;
Detektieren einer Position des OOI basierend auf der Richtungsinformation des Fötus, der Breitenmittellinie und der Höhenmittellinie; und
Erzeugen von Positionsinformationen des detektierten OOI basierend auf der detektierten Position des OOI.

8. Verfahren nach Anspruch 7, welches des Weiteren das Speichern einer Vielzahl von Symbolen aufweist, welche eine Vielzahl von Fötushaltungen bzw. -lagen und Richtungsinformationen eines Fötus entsprechend jeder der Vielzahl von Symbolen anzeigt.

9. Verfahren nach Anspruch 8, wobei der Benutzerbefehl Folgendes aufweist:
einen ersten Benutzerbefehl zum Anzeigen der Vielzahl von Symbolen auf dem Bildschirm; und
einen zweiten Benutzerbefehl zum Auswählen eines Symbols aus der Vielzahl von Symbolen.

10. Verfahren nach Anspruch 9, welches des Weiteren Folgendes aufweist:
Anzeigen der Vielzahl von Symbolen auf dem Bildschirm als Reaktion auf den ersten Benutzerbefehl; und
Festlegen von Richtungsinformationen entsprechend dem ausgewählten Symbol auf dem Bildschirm als Reaktion auf den zweiten Benutzerbefehl.

## Revendications

1. Système ultrasonique (100) comportant :
une unité (110) d'acquisition de données agencée pour transmettre un signal ultrasonique sur un objet
comprenant un foetus, et pour acquérir des données ultrasoniques en recevant un signal d'écho ultrasonique réfléchi par l'objet ;
un processeur(130) agencé pour générer
une image ultrasonique avec les données ultrasoniques;
un affichage (150) adapté pour afficher l'image ultrasonique sur un écran ; et
une unité d'entrée utilisateur (120) agencée pour recevoir
une commande de l'utilisateur pour fixer des informations de position du foetus correspondant à une posture du foetus basée sur l'image ultrasonique,
dans lequel le processeur (130) est agencé pour définir les informations de position indiquant la posture
du foetus et incluant au moins une information relative à l'avant, l'arrière, la droite et la gauche du foetus, sur l'écran en réponse à la commande de l'utilisateur, **caractérisé en ce que** le processeur est agencé pour détecter un objet d'intérêt
(OOI) de l'image ultrasonique affichée sur l'écran avec les informations de direction du foetus telles que définies, détecter un point central de l'image ultrasonique, définir une ligne centrale en largeur et une ligne centrale en hauteur par rapport au point central sur l'image ultrasonique, détecter une position du OOI basée sur les informations de direction du foetus, la ligne centrale en largeur et la ligne centrale en hauteur et générer des informations de position du OOI détecté basées sur la position détectée du OOI.

2. Système ultrasonique (100) selon la revendication 1, comprenant en outre une mémoire (140) agencée pour mémoriser
une pluralité d'icônes indiquant une pluralité de postures et d'informations de directions de foetus correspondant à chacune de la pluralité d'icônes.

3. Système ultrasonique (100) selon la revendication 2, dans lequel la commande utilisateur inclut :
une première commande d'utilisateur pour afficher la pluralité d'icônes sur l'écran de l'affichage (150) ; et
une seconde commande d'utilisateur pour sélectionner une icône parmi la pluralité d'icônes.

4. Système ultrasonique (100) selon la revendication 3, dans lequel le processeur (130) est agencé pour afficher
la pluralité d'icônes sur l'écran de l'affichage (150) en réponse à la première commande d'utilisateur et déterminer des informations de direction, correspondant à l'icône sélectionnée sur l'écran de l'affichage (150) en réponse à la seconde commande d'utilisateur.

5. Système ultrasonique (100) selon la revendication 1, dans lequel le processeur (130) est agencé pour générer
des informations additionnelles pour indiquer un degré de proximité selon lequel le OOI s'approche du point central vers l'information de position du foetus, basée sur l'information de direction du foetus.

6. Système ultrasonique (100) selon la revendication 1, dans lequel le OOI inclut un coeur du foetus.

7. Procédé pour créer une information de position d'un objet en utilisant le système ultrasonique (100) de la revendication 1,
ce procédé comportant :
la transmission d'un signal ultrasonique sur un objet comprenant un foetus, et l'acquisition de données ultrasoniques en recevant un signal d'écho ultrasonique réfléchi par l'objet ;
la génération d'une image ultrasonique avec les données ultrasoniques;
l'affichage de l'image ultrasonique sur un écran ;
la réception d'une commande de l'utilisateur pour fixer des informations de position du foetus correspondant à une posture du foetus basée sur l'image ultrasonique ;
et la détermination d'informations de position indiquant la posture du foetus et incluant au moins une information relative à l'avant, l'arrière, la droite et la gauche du foetus, sur l'écran en réponse à la commande de l'utilisateur ;
**caractérisé par**
la détection d'un objet d'intérêt (OOI) de l'image ultrasonique affichée sur l'écran avec les informations de direction du foetus telles que définies :
la détection d'un point central de l'image ultrasonique, la détermination d'une ligne centrale en largeur et
d'une ligne centrale en hauteur par rapport au point central sur l'image ultrasonique :
la détection d'une position du OOI basée sur les informations de direction du foetus, la ligne centrale en largeur et la ligne centrale en hauteur ; et
la génération des informations de position du OOI détecté basées sur la position détectée du OOI.

8. Procédé selon la revendication 7, comprenant en outre une pluralité d'icônes indiquant une pluralité de postures et d'informations de directions de foetus correspondant à chacune de la pluralité d'icônes.

9. Procédé selon la revendication 8, dans lequel la commande d'utilisateur inclut :
une première commande d'utilisateur pour afficher la pluralité d'icônes sur l'écran ; et une seconde commande d'utilisateur pour sélectionner une icône parmi la pluralité d'icônes.

10. Procédé selon la revendication 9, comprenant en outre :
l'affichage de la pluralité d'icônes sur l'écran en réponse à la première commande d'utilisateur ; et
la détermination des informations de direction correspondant à l'icône sélectionnée sur l'écran en réponse à la seconde commande d'utilisateur.
